# EUROPEAN PATENT APPLICATION

(11) **EP 1 210 921 A2**
(43) Date of publication of application: **05.06.2002**
(21) Application number: 01204689.2
(22) Date of filing: 04.12.2001
(51) Int. Cl.: A61F 5/41

(54) **Device for assisting erectile function in men**

(30) Priority: 04.12.2000 NL 1016792
(71) Applicant: Van Velzen Cornelis Leonardus Maria, 3421 CD Oudewater (NL)
(72) Inventor: Van Velzen Cornelis Leonardus Maria, 3421 CD Oudewater (NL)
(74) Representative: Huygens, Arthur Victor

(57) **Abstract**

A device (1) is provided for assisting erectile function in men which comprises a resilient non-continuing ring-shaped element (2), at the ends provided with fastening means (3) and having an inner and outer diameter, wherein the inner diameter is selected such that upon sexual stimulation the element causes an external pressure on the penis and a change of internal pressure sufficient to increase the pressure in the cavernous bodies and/or to maintain the increased pressure, wherein the device (1) is substantially made of elongatable material. In a preferred embodiment the device is provided with a locking element limiting the elongation of the elongatable material. The fastening means (3) are fixed preferably at the ends (5) of the locking element.

## Description

The present invention relates to a device for assisting erectile function in men, comprising a flexible non-continuing ring-shaped element, provided at their ends with fastening means, and having an inner and outer diameter, wherein the inner diameter is selected such that upon sexual stimulation the element causes an external pressure on the penis and a change of internal pressure sufficient to increase the pressure in the cavernous bodies and/or to maintain the increased pressure, wherein the device is substantially made of elongatable material.

European Patent 0 586 621 reports that about 5% of men in the 40th year of life and 20% in the 60th year of life suffer from an erectile dysfunction. The loss of potency is a shock to the man's, in particular the young man's view of himself in physical, emotional and social terms. Men with chronic erectile dysfunction are unsure of their sexuality and personality, with all consequences thereof.

U.S. Patent No. 5,513,652 describes in the introduction that normal erectile function requires numerous physiologic events to occur in concert. First, adequate neuropsychogenic. chemical or electrical stimulus must be present. Second, there must be adequate arterial inflow into the penis. Third, the corporal, smooth muscles must relax and corporal epithelial tissue must respond to the erectile stimulus, thus allowing the corporal sinusoids to expand and fill with blood. Finally, the venous closure mechanism must be initiated to prevent outflow of blood, thus resulting in storage of blood within the cavernous bodies ("corpora cavernosa") which are responsible for erections. Each of the cavernous bodies is supplied by a cavernosal artery. The cavernosal artery flow and pressure determine the arterial competence of the erectile process. As the blood fills and expands these hydraulic chambers the corporal venules and penile veins are compressed. This compression increases outflow resistance, permitting blood to flow into but not out of the cavernous bodies. Intracorporal resistance is usually between about 5 to 10 mm Hg x min/ml. Tumescence and rigidity results as blood is increasingly stored within the cavernous bodies. Early in tumescence, intracorporal pressure is about 10 mm Hg. During full tumescence, intracorporal pressure is between about 90 and 150 mm Hg. Borderline erectile function is available at about 50 mm Hg. Detumescence occurs when arterial inflow decreases, or corporal outflow increases, resulting in a net loss of blood from the penis.

Some men are unable to obtain or sustain an adequate erection because sufficient intracorporal pressure cannot be achieved or maintained.

Various solutions to this problem have been mentioned in the literature, which are all based on external devices. For example, Dutch patent 1002838 discloses an erection aid comprising a pivoting non-continuous ring with fastening means, the ring being fixed in its open state by encircling the base of a penis and scrotum at the junction of the pubis, then closing the pivoting ring and securing it using a hook. U.S. Patent No. 5,027,800 discloses a kit for a man's erection truss requiring final fitting and completion by the man, with or without assistance. When completed, this erection truss is essentially a completed loop of surgical tubing, or the like, having a male-female, preferably plastic, in line encirclement fastener assembly, removably fastened, which is glued on each of the cut ends of the tubing. U.S. Patent No. 5,085,209 discloses a device consisting of a band of material which is formed as a collar around the penis in one circle and having portions comprised of an interlocking material, e.g. Velcro®, to interconnect against itself when so encircled, and further including a loop extending from an end of the interlocking material. The device is cinched as tightly as possible around the base of the penis and scrotum at their junction with the pubis by use of the loop located at one end of the device through which the other end is inserted, pulled and interlocked. U.S. Patent No. 5,855,548 discloses a tubular device including an integral fastening means adapted to hold and secure the tubing in a loop configuration about the penis to provide an adjustable radial constrictive force about the base of the penis. The integral fastening means is provided in part by a substantially rigid, enlarged segment of tubing adjacent to the proximal end of the tubing. The enlarged segment of tubing operates in combination with an annular ring to provide a ball and coupling configuration. A similar solution with an adjustable holding means fixed on both ends of a device of a wound elongated member is described in German Patent No. 44 03 103. Furthermore, WO 92/17136 discloses a ring-shaped venous constrictor device in the form of a flat tension band equipped with friction means and primary holding means in the partly wound state. Fully tensioned band, when in place, has a secondary holding means.

None of these solutions is satisfactory in actual practice, both because the fitting of the devices is complicated and time-consuming and/or the devices are not comfortable.

It is an object of the present invention to solve this problem or at least to contribute considerably to its solution by providing a device which can be easily fixed and, moreover, is not inconvenient when used.

The invention is therefore characterised in that the flexible non-continuing ring-shaped element is made of elongatable material.

In a preferred embodiment the device further comprises a locking element which restricts the elongation of the elongatable material. In a further preferred embodiment the fastening means comprises a hook and eye or two hooks arranged to the respective ends of the device.

The ring-shaped element is fixed around the penis behind the scrotum and is preferably closed at the upper side by fixing together the hook and eye or two hooks, respectively. Preferably, the fastening element of the device should be detached fairly easily, which is attained by the mechanisms described above.

Preferably, the elongatable, flexible ring-shaped element of the invention is substantially made of rubber, preferably foamed silicone rubber, metal or plastic material. The material preferably is tubular, generally having a relatively thick wall, so that the locking element which is of a greater length than the elongatable material in its non-elongated state, can be easily fixed and stored and moved in the internal cavity. For example, a suitable tubular material of foamed silicone rubber in its non-elongated state has an outer diameter ranging between 6.5 to 9.5 mm, preferably 7.5 to 8.5 mm. The inner diameter can also vary and generally ranges between 0.5 and 3 mm, preferably between 1 and 2 mm.

The locking element is, for example, a thin piece of string substantially made of non-elongatable material, such as nylon, of a predetermined size. Generally, the piece of string is guided through the flexible ring-shaped element. In a preferred embodiment the fastening means is arranged on the respective ends of the piece of string. The length of the piece of string is generally 5 to 10 cm longer than the flexible ring-shaped material in non-elongated state. The far ends of the elongatable, flexible, ring-shaped element are suitably fixed adjacent to or at the ends of the piece of string, for example by a suitable adhesive, such as a glue.

Preferably, the element has a predetermined length which is selected such that the external pressure of the device on the penis in erected state is not too strong, which would be experienced as unpleasant by the user and which in the extreme may cause serious strangulation. In that case the blood will also undergo great resistance in the inflow into the cavernous bodies. In the preferred embodiment mentioned above the size of the device is determined predominantly by the length of the locking element which generally passes through the ring-shaped element. A person skilled in the art of materials will not have any difficulty in selecting and adapting the most appropriate materials for the intended application, realising that the elongation and resilience of the material are essential elements.

The length of the elongatable, flexible ring-shaped element according to the invention is not unequivocal and is generally determined by individual factors. In order to realise the proper functioning of the device according to the invention, accurate measuring by the user or a physician is important, so that the device fits well, not too loose and not too tight, otherwise the desired effect described above will not or insufficiently occur, or just too largely. The measurements of the device, i.e. the ring-shaped element and the piece of string passing through the ring-shaped element, respectively, are preferably taken with the penis in detumescent (relaxed) state by measuring the outline of the base of the penis and scrotum at the junction of the pubis, behind the scrotum and around the upper side of the penis. The device according to the invention including an elongatable, flexible ring-shaped element and a piece of string is therefore provided in different sizes, preferably varying 0.5 cm between them. Suitable lengths, in elongated state, include, for example, 10. 10.5, 11, 11.5, 12, 12.5, 13, 13.5 14, 14.5, 15, 15.5, 16 cm., and so on.

According to another embodiment of the invention the ring-shaped element consists essentially of a coiled spring, generally made of spring steel. It was found that a round coiled tension spring made of steel, having a thickness of 0.5-0.7 mm and an outer diameter of about 6 mm, has a proper tension and offers sufficient user comfort.

The elongatable, flexible ring-shaped element according to the invention may further be coated by a cover, preferably of thin pliable plastic material, in particular when the ring-shaped element is made of metal. The cover is usually fixed to the far ends of the ring-shaped element, at or adjacent to the fastening means. The length of the cover is about 5 cm longer than the length of the ring-shaped element (in non-elongated state, so that the cover does not affect the resilient action of the ring-shaped element). Optionally, the ring-shaped element itself can also be provided with a suitable cover, for example a plastic foil which is put on a coiled tension spring made of steel.

As mentioned before, the action of the device according to the present invention is based in principle on longer maintaining the increased tumescence of the penis upon sexual stimulation resulting in a more intense and longer lasting erection, thus permitting penetration, possibly several times. Moreover, the elasticity of the ring-shaped element will also prevent a too rapid ejaculation.

Fitting the device is very simple and can be achieved by the resilience of the material both in erected and non-erected state of the penis. Both ends of the element are firmly taken between thumb and forefinger and the element is brought under the penis behind the scrotum. Both ends are then fastened at the upper side of the penis, or in an alternative way, if desired.

The device is hardly felt in relaxed state, so that it can be borne for a prolonged period of time without problems. Because of the resilient character of the ring-shaped element serious strangulation will never occur. The ring can be reused many times under normal conditions without losing its elasticity, which makes this device very cheap relative to similar devices belonging to the state of the art.

The ring has been tested by various test persons with good result. The test persons told that their self-confidence had been increased. No disadvantages or side-effects were mentioned.

The present invention is now further illustrated with reference to the accompanying drawings which are not intended to limit the scope of the invention in any respect. Reference signs to the same or similar elements in the various drawings are identical.

Figure 1 is a diagrammatic cross-section of a device according to the present invention showing a flexible ring-shaped means in a non-elongated state, with fastening means, in a closed state.

Figure 2 is a view of the device of Figure 1 in an open non-elongated state.

Figure 3 shows an embodiment in which the device is equipped with a piece of string as a locking device and the fastening means fixed to the far ends of the piece of string. For the sake of clarity the elongatable, flexible ring-shaped element is not fixed here to the far ends of the piece of string.

Figure 4 is a view of an alternative device according to the invention in the form of a coiled tension spring with cover, in a closed state.

Figure 5 is a view of the covered coiled spring of Figure 4, but in an open, extended, but not-elongated state.

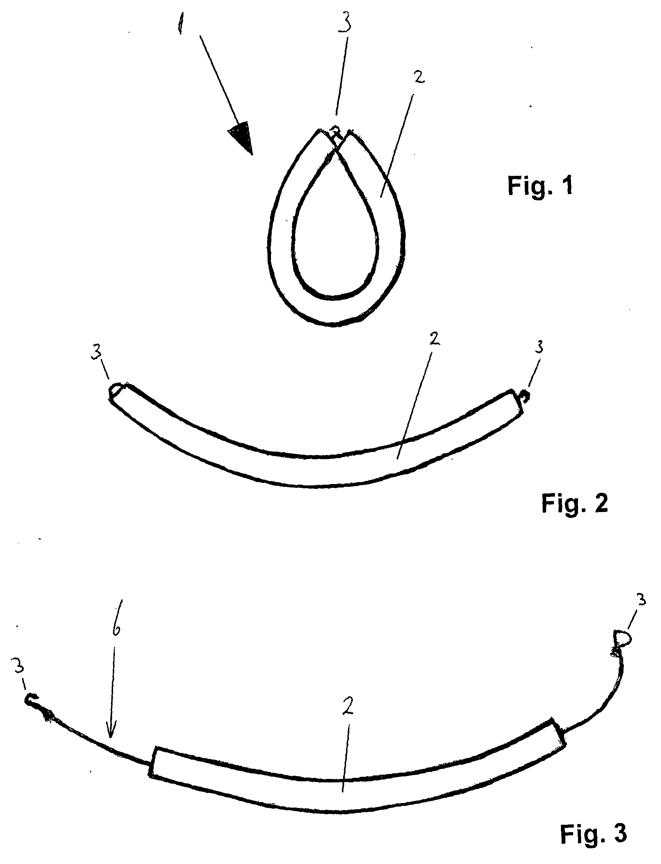

Figures 1 and 2 show the device **1** according to the invention, comprising essentially an elongatable flexible ring-shaped element **2,** with a fastening assembly **3.** The fastening mechanism is very simple and effective, and is illustrated here by a hook and eye which are arranged to the respective ends of the ring-shaped element. Alternative fastening means will be apparent to a skilled person and may for example consist of two engaged hooks or eyes, or a screw connection. In the embodiment of Figures 1 and 2 the flexible ring-shaped element **2** consists of elongatable, tubular material of foamed silicone rubber having a relatively thick wall which was found to possess the desired properties as defined above.

Figure 3 shows a preferred embodiment in which the device **1** comprises a locking element **6**, consisting of a piece of string, for example of nylon or similar substantially non-elongatable material. Here the fastening means consisting of a hook and eye, or similar means, is fixed to the far ends of the locking element **6**. The ends of the flexible ring-shaped element **2** are usually fixed to or near the far ends of the locking element (not shown for the sake of clarity), and the greater length of the piece of string of the locking element relative to the ring-shaped element in non-elongated state is stored in the inner part of the ring-shaped element **2**. When this ring-shaped element **2** is elongated during use, the piece of string of the locking element **6** becomes extended until its maximum length is reached and the device exerts its action at the maximum elongated state with an erected penis, as described above. It is noted that the embodiment of Figure 3 in closed form has a similar view as the embodiment of Figures 1 and 2. The only difference is, that in Figures 1 and 2 the fastening means **3** is fixed to the far ends of the ring-shaped element **2**, whereas in Figure 3 the fastening means **3** is fixed to the far ends of the locking element **6**.

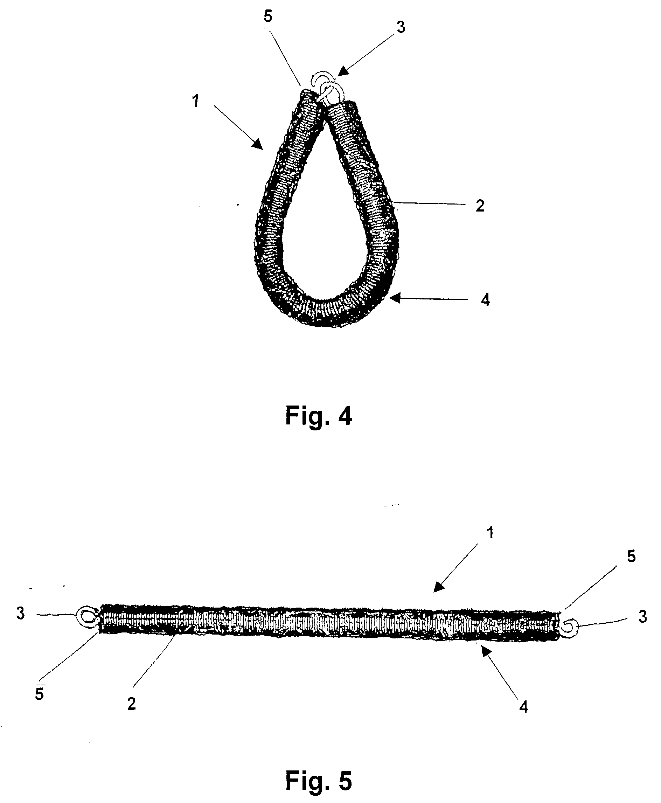

The flexible ring-shaped element **2** in the embodiment of Figures 4 and 5 is a coiled tension spring made of steel. The thickness of the spring is about 0.6 mm here and its outer diameter about 6 mm, but these values are variable depending on the wishes and needs of the user. These values can be further optimized, if desired. The mentioned values appeared to result to excellent results in practice. The length of the spring is also variable and is mainly determined by the individual dimensions of the particular users. The spring may also be equipped by a locking element (not shown), similar to the embodiment of Figure 3. In the embodiment shown in Figs. 4 and 5 the length of the spring in extended form is 11.0 cm without the fastening means and 12.0 cm with the fastening means. Around the ring-shaped element **2** a wide cover **4** of plastic material may be arranged which is fixed to the spring at the two far ends **5**.

The device according to the invention is suitable for any sexually active men, both adolescents and adults. However, a particular target group is formed by men having an erectile dysfunction.

## Claims

1. A device for assisting erectile function in men which comprises a resilient non-continuing ring-shaped element, provided at the ends with fastening means and having an inner and outer diameter, wherein the inner diameter is selected such that upon sexual stimulation the element causes an external pressure on the penis and a change of internal pressure sufficient to increase the pressure in the cavernous bodies and/or to maintain the increased pressure, wherein the device is substantially made of elongatable material.

2. The device as claimed in claim 1, further comprising locking means limiting the elongation of said elongatable material.

3. The device as claimed in claim 1 or 2, **characterised in that** said flexible ring-shaped element is substantially made of rubber, metal or plastic.

4. The device as claimed in claim 3, **characterised in that** said flexible ring-shaped element is made of foamed silicone rubber.

5. The device as claimed in any one of claims 1 to 4, **characterised in that** said flexible ring-shaped material is hollow.

6. The device as claimed in claim 3, **characterised in that** said flexible ring-shaped element is a coiled tension spring made of steel.

7. The device as claimed in any one of the preceding claims, **characterised in that** the fastening means comprises a hook and eye or two hooks.

8. The device as claimed in any one of claims 2 to 7, **characterised in that** the locking element comprises a piece of string which passes through said elongatable, flexible ring-shaped element.

9. The device as claimed in any one of claims 2 to 8, **characterised in that** said fastening means is fixed to the ends of the locking element.

10. The device as claimed in any one of claims 1 to 9, **characterised in that** the elongatable, flexible ring-shaped element is provided with a cover.
